# EUROPÄISCHE PATENTSCHRIFT

(11) **EP 1 143 939 B1**
(45) Veröffentlichungstag und Bekanntmachung des Hinweises auf die Patenterteilung: **13.08.2008**
(21) Anmeldenummer: 00958444.2
(22) Anmeldetag: 14.08.2000
(51) Int. Cl.: A61K 9/70

(54) **WIRKSTOFFHALTIGES THERAPEUTISCHES SYSTEM ZUM AUFTRAG AUF DIE HAUT MIT DREI POLYMERHALTIGEN SCHICHTEN**
THERAPEUTIC SYSTEM CONTAINING AN ACTIVE SUBSTANCE FOR THE APPLICATION ON THE SKIN WHICH CONTAINS THREE POLYMEROUS LAYERS
SYSTEME THERAPEUTIQUE AVEC AGENT ACTIF DESTINE A UNE APPLICATION EPIDERMIQUE COMPORTANT TROIS COUCHES CONTENANT DES POLYMERES

(30) Priorität: 25.08.1999 DE 19940238
(43) Veröffentlichungstag der Anmeldung: 17.10.2001
(73) Patentinhaber: LTS LOHMANN Therapie-Systeme AG, 56626 Andernach (DE)
(72) Erfinder: BERTHOLD, Achim, 56626 Andernach (DE)
(74) Vertreter: Flaccus, Rolf-Dieter
(86) Internationale Anmeldenummer: PCT/EP2000/007900
(87) Internationale Veröffentlichungsnummer: WO 2001/013899

(56) Entgegenhaltungen:
- EP-A- 0 464 573
- DE-A- 3 231 400
- DE-C- 19 706 824

## Beschreibung

Die Erfindung betrifft ein wirkstoffhaltiges therapeutisches System zur Applikation auf der Haut mit drei aufeinander laminierten matrixschichten . Weiterhin betrifft die Erfindung ein Herstellungsverfahren sowie eine Verwendung des therapeutischen Systems.

Mit einer pharmazeutischen Zubereitung zur Abgabe von Wirkstoffen an die Haut wird entweder eine transdermale systemische Wirkung oder eine dermale, lokale Wirkung der abgegebenen Wirkstoffe angestrebt. Das Anhaften auf der Applikationsfläche wird durch Kleber sichergestellt, welche hochviskose, permanent klebende Strukturen ausbilden. Dabei werden Qualitätsmerkmale wie Anfangsklebrigkeit (Tack), Haftvermögen (Adhäsion) und die innere Festigkeit des Haftklebers (Kohäsion) unterschieden.

Die pharmazeutische Zubereitung ist eine wirkstoffhaltige Vorrichtung, die einen oder mehrere Arzneistoffe in einer vorausbestimmbaren Rate kontinuierlich über einen festgelegten Zeitraum an einem festgelegten Ort freigibt. Eine derartige Vorrichtung ist durch ein exaktes Behandlungsprogramm, als Dosierungsprogramm bezeichnet, charakterisiert und wird als therapeutischen System (TS) bezeichnet. Da erfindungsgemäße Systeme als Pflaster auf die Haut geklebt werden, um entweder einen systemischen oder einen lokalen Effekt zu erzielen, spricht man in diesem Zusammenhang von transdermalen therapeutischen Systemen (TTS) oder von dermalen therapeutischen Systemen (DTS).

Die erfindungsgemäße Zubereitung hat einen hohen Wirkungsgrad. Das heißt, die Zubereitung führt zu einer hohen Bioverfügbarkeit von Wirkstoffen. Die Wirkung kann durch einfache Entfernung der Zubereitung jederzeit abgebrochen werden. Infolgedessen zeichnet sich die Zubereitung auch durch eine Steuerbarkeit der Wirkstoffabgabe aus. Die erfindungsgemäße Zubereitung hat weiterhin eine hohe Zuverlässigkeit im Hinblick auf die Befolgung des Therapieplans durch den Patienten (Patientenakzeptanz, sog. compliance), da die Applikationsfrequenz im Vergleich zu konventionellen Arzneiformen stark reduziert ist und Nebenwirkungen selten in Erscheinung treten. Ferner kann die applizierbare Wirkstoffmenge in der Regel vermindert werden. Somit werden dosisabhängige Nebenwirkungen ebenfalls reduziert oder vermieden. Daraus resultiert eine erhöhte Therapiesicherheit.

Üblicherweise umfaßt ein wirkstoffhaltiges System, TTS oder DTS, einen Aufbau mit mehreren Schichten, umfassend wenigstens eine Wirk- und/oder Hilfsstoffe enthaltende Reservoirschicht, eine für deren Inhaltsstoffe impermeable Rückschicht und eine vor Applikation auf der Haut abzulösende Schutzschicht.
Dabei besteht die Reservoirschicht in aller Regel aus einem Wirk- und/oder Hilfsstoff enthaltenden amorphen Polymer. Neben einer Anzahl vorteilhafter Eigenschaften des Polymers, insbesondere im Hinblick auf ihre Verwendung als Pflaster, wie diffusionsbedingte Aufnahme oder Abgabe der Inhaltsstoffe, oder ihre Flexibilität bei Anpassung an eine vorhandene Körperform am Applikationsort, neigen andererseits amorphe Polymere infolge vergleichsweise nicht ausreichender Kohäsion besonders bei längerer Lagerung im Pflaster zu kaltem Fluß.

Während bei rein kristallinen Stoffen die Molekualarbewegung mit steigender Temperatur beim Schmelzpunkt sprunghaft von einem relativ niedrigen Niveau auf ein hohes Niveau ansteigt, verhalten sich amorphe Polymere, wie beispielsweise Kleber, anders.
Die Molekularbewegung nimmt mit steigender Temperatur in mehreren, meist 5 verschiedenen, reversiblen Stufen zu. Im einzelnen werden fünf Viskoelastizitätsbereiche als Glaszustand (hart; niedrigste Temperatur), Lederzustand (Wendepunkt = Glasübergangstemperatur (*Tg*) = Temperatur, bei der ein Polymer vom festen Glaszustand in den kautschukelastischen Zustand übergeht), kautschukelastischer Zustand, kautschukelastisches Fließen und viskoser Zustand (höchste Temperatur) bezeichnet.
Der Übergang vom Glaszustand in den kautschukartigen Zustand ist begleitet von einer deutlichen Änderung der physikalischen Eigenschaften wie spezifisches Volumen, Elastizitätsmodul, Wärmekapazität, thermomechanische Eigenschaften und Brechungsindex. Der Schmelzpunkt (*Tm*; festes und flüssiges Polymer im Gleichgewicht) amorpher Polymere ist durch teilkristalline Polymerbereiche definiert. Hierbei liegt *Tg* immer niedriger als *Tm*.

### Man unterscheidet:

Polymere, die sich bei Raumtemperatur (*Ta*) im festen Glaszustand befinden, mit *Tg, Tm* > *Ta*. Beispiele sind isotaktisches Polystyrol: *Tg* = 85°C / *Tm* = 240 °C; Polyethylenterephthalat: *Tg* = 69°C / *Tm* = 265°C; Polyhexamethylenadipamid: *Tg* = 53°C / *Tm* = 265 °C; Polytetrafluorethylen: *Tg* = 126 °C / *Tm* = 325 °C) .
Polymere, die sich im weichen kautschukähnlichen Zwischenzustand befinden, (*Tm* > *Ta* > *Tg*). Beispiele sind Niederdruckpolyethylen: *Tg* = -70 °C / *Tm* = 139 °C; isotaktisches Polypropylen: *Tg* = -18 °C / *Tm* = 186 °C .
Polymere, die sich im flüssigen viskosen Zustand befinden (*Ta > Tm*, *Tg;* Beispiele sind Polydimethylsiloxan: *Tg* = -121 °C / *Tm* = -40 °C; 1,4-cis-Polybutadien: *Tg* = -95°C / *Tm* = 2 °C).

Hieraus läßt sich ableiten, daß die Kohäsion und somit kalter Fluß eines Klebers mit hochviskosen, permanent klebenden Polymerstrukturen ausschlaggebend von der Glasübergangstemperatur der eingesetzten Polymere beeinflußt wird.

Kalter Fluß umschreibt eine Materialeigenschaft. Die davon betroffenen Materialien beginnen während der Lagerung zu fließen ohne speziellen Einflüssen ausgesetzt zu sein und können demzufolge als hochviskose Flüssigkeiten betrachtet werden.

Es ist aus der Praxis bekannt, daß TTS und DTS oftmals zu kaltem Fluß neigen. Dieser führt dazu, daß ein Pflaster während seiner Lagerung mit dem Primärpackmittel verklebt und nachfolgend nur noch schwer entnommen werden kann. Ein weiteres Problem besteht darin, daß Pflaster mit kaltem Fluß nach ihrer Applikation schwarze Ränder, Kleberrückstände, auf der Applikationsfläche hinterlassen, die sich teilweise nur unter intensiven Reinigungsmaßnahmen entfernen lassen.

WO 86/00814 sowie US 5,186,938 beschreiben die Möglichkeit, kalten Fluß von Glyceroltrinitrat enthaltenden, selbstklebenden, von Polyacrylsäure abgeleiteten Polymeren dadurch zu verbessern, daß die Polymere quervernetzt werden. Zur Quervernetzung werden beispielsweise divalente Metallionen oder Melamin eingesetzt. Dadurch entsteht ein kohärentes Netzwerk, welches wesentlich weniger zu kaltem Fluß neigt als das Ausgangspolymer. Eine Vernetzung, die sich positiv auf die Kohäsion auswirkt, hat aber einen negativen Einfluß auf die Klebrigkeit, welche sich dadurch verschlechtert.

Letzteres ist eine allgemeingültige Faustregel. Die Erfinder des bekannten Verfahrens versuchen das Problem dadurch zu lösen, daß sie das Agens, welches die Quervernetzung bewirkt, nur in einer relativ geringen Menge einsetzen, um eine Mindestklebrigkeit zu gewährleisten. Das Problem an der geschilderten vorgehensweise besteht darin, daß der Grad der Quervernetzung exakt eingestellt werden muß, um optimale Systemeigenschaften zu gewährleisten.

Um nicht von der durch den Grad der Quervernetzung bedingten Empfindlichkeit der Kohäsion und Klebrigkeit abhängig zu sein, wird mit EP 0 856 311 A1 und DE 197 06 824 C1 eine andere Vorgehensweise vorgeschlagen. Um die Kohäsion der Matrix zu verbessern, ohne die Klebkraft auf der Haut zu reduzieren, werden Systeme vorgeschlagen, die aus mindestens zwei Schichten bestehen. Das Besondere hierbei ist, daß die Schichten zwar die gleiche Polymerzusammensetzung und die gleiche Konzentration an gelösten Inhaltsstoffen aufweisen, sich aber im Grad ihrer Quervernetzung unterscheiden. Während die der Haut augewandte Schicht, die einen niedrigere Quervernetzungsgrad aufweist, die Hauthaftung gewährleistet, reduziert die darüber liegende Schicht mit höherem Quervernetzungsgrad den kalten Fluß. Die Quervernetzung erfolgt in bekannter Weise, beispielsweise durch Zusatz von Metallionen oder reaktiven Reagenzien sowie durch Elektronenbestrahlung oder Bestrahlung mit UV-Licht.

Eine andere Möglichkeit zur Verbesserung der Kohäsion ist aus DE 40 20 144 C2 bekannt. Diese beschreibt eine Möglichkeit, den kalten Fluß ohne Einsatz von Reagenzien zur Quervernetzung zu verbessern. Hierzu wird dem selbstklebenden Basispolymer ein weiteres, nichtklebenden, aber filmbildendes Polymer zugesetzt. Das filmbildende Polymer, welches in der Regel durch ein hohes Molekulargewicht charakterisiert ist, ergibt eine wesentliche Verbesserung der Kohäsion des Systems.

DE 32 31 400 A1 offenbart ein selbsthaftenden medizinisches Verbundpräparat, das eine selbstklebende Schicht einer makromolekularen Substanz und eine dieser Schicht benachbarte Polymerisatschicht umfaßt, bei dem eine Kristallisation des Arzneimittels in der Schicht der makromolekularen Substanz durch Einarbeiten eines Adjuvans in das Verbundpräparat verhindert wird, und bei dem eine verbesserte perkutane Absorption, Diffusion und Löslichkeit des Arzneimittels erreicht werden.

Die Glasübergangstemperatur der Schicht einer makromolekularen Substanz wird mit -70°C bis -10°C angegeben, die Polymerisatschicht soll ein Polymerisat enthalten, das eine Glasübergangstemperatur aufweist, die nicht niedriger als -50°C liegt. Jedoch kann die Polymerisatschicht bis zu 90 Gew.-% andere Bestandteile enthalten, die keine Glasübergangstemperatur haben oder deren Glasübergangs-temperatur nicht spezifiziert ist, so daß die Glasübergangstemperatur der Polymerisatschicht als solche nicht offenbart wird.

Mit der EP 0 464 573 A1 wird ein vorrichtung zur topischen und systemischen Verabreichung von Wirkstoffen an bzw. durch die Haut beschrieben, die aus einer Rückschicht, mindestens einer selbstklebenden Schicht und einer wieder entfernbaren Schutzschicht besteht, wobei die selbstklebende Schicht aus 100 Gewichtsteilen eines Polyacrylatklebers, 5 bis 150 Gewichtsteilen eines mit Polyacrylaten verträglichen Filmbildners, 0 bis 250 Gewichtsteilen nicht weichmachendem Wirk- und/oder Hilfsstoff und 10 bis 250 Gewichtsteilen weichmachendem Wirk- und/oder Hilfsstoff besteht.

In EP 0 464 573 A1 werden weder Glasübergangstemperaturen für die selbstklebende Schicht angegeben, noch wird das Problem des kalten Flußes angesprochen.

Die beschriebenen Verfahren zur Verbesserung der Kohäsion sind nur bedingt anwendbar. Beispielsweise sind nicht alle klebenden und damit zur Herstellung von selbstklebenden TTS bzw. DTS geeigneten Polymere quervernetzbar. Auch die Zumischung von kohäsionsverbessernden Polymeren ist aus Kompatibilitätsgründen nicht immer möglich.

Ausgehend vom vorgenannte Stand der Technik liegt der Erfindung die Aufgabe zugrunde, ein Verfahren zur Verbesserung der Kohäsion zwecks deutlicher Reduzierung des kalten Flußes bereitzustellen, welches die bisher beobachteten Schwierigkeiten und technischen Grenzen wenigstens größtenteils überwindet und zu einer hohen Bioverfügbarkeit der enthaltenen Wirk- und Hilsstoffe führt.

Die Aufgabe wird gemäß Anspruch 1 durch einen geschichteten Aufbau des TTS bzw. DTS gelöst.

Hierbei unterscheiden sich die verschiedenen Schichten durch ihre Glasübergangstemperatur (Tg). Die Schicht (en) mit der (den) höheren Glasübergangstemperatur(en) führt (führen) zu einer Verbesserug der Kohäsion des gesamten Systems. Demzufolge ist der kalte Fluß reduziert, so daß das Problem, daß die TTS bzw. DTS während der Lagerung mit dem Primärpackmittel verkleben sowie nach ihrer Applikation schwarze Ränder infolge Kleberrückständen auf der Applikationsfläche hinterlassen, nicht mehr in Erscheinung tritt bzw. stark reduziert ist. Ferner werden Inkompatibilitäten dadurch vermieden, daß die eingesetzten Polymere in unterschiedlichen Schichten vorliegen und so ihre Interaktion im wesentlichen auf die Grenzflächen beschränkt bleibt. In günstigen Fällen kann dabei auf eine Quervernetzung verzichtet werden.

Weitere Ausgestaltungen der Erfindung sind entsprechend den Unteransprüchen vorgesehen.
Dabei kann eine der Schichten zugleich als Steuerungsmittel für die Wirkstoffreigabe ausgebildet sein.
Weiterhin sieht das Verfahren nach der Erfindung vor, daß zumindest eine der Schichten als Wirkstoffreservoir ausgebildet und angeordnet ist.

Schließlich umfaßt ein Verfahren zur Herstellung des therapeutischen Systems die Schritte, daß drei polymerhaltige Schichten aufeinander laminiert werden, wobei sich die für die mittlere Matrixschicht und die für die äußeren Matrixschichten eingesetzten Polymers in ihrer Glas-übergangs temperatur unterscheiden
Eine Verwendung des therapeutischen Systems nach der Erfindung dient zur topischen oder transdermalen Abgabe von Wirkstoffen an die Haut eines Organismus.

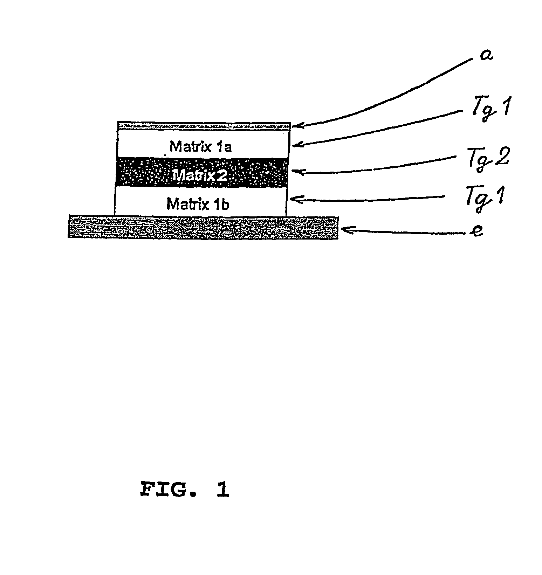

Eine beispielhafte, schematische Darstellung des erfindungsgemäßen Systems ist aus der Figur 1 erkennbar.
Darin ist ein Ausführungsbeispiel gezeigt, welches aus fünf Schichten (a bis e) besteht. Die Schichten sind:
a) Rückschicht
b) Matrix 1a mit einem als Tg1 gekennzeichneten Polymer,
c) Matrix 2 mit einem als Tg2 gekennzeichneten Polymer,
d) Matrix 1b mit einem als Tg1 gekennzeichneten Polymer,
e) Schutzschicht, ablösbar, wobei Tg2 > Tg1 ist.

### Beispiel:

Zur Bereitung der Matrix 1a wurden 25,0 g eines Polymers auf der Basis der Methacrylsäure (Eudragit^{®} L100) in 16,1 g Ethanol gelöst. Nach der vollständigen Auflösung des Methacrylsäurepolymers wurden zu dieser Vorlösung die Wirkstoffe Estradiol (925,0 mg) und Norethisteronacetat 5,25 g) gegeben und erneut gerührt, bis die Wirkstoffe aufgelöst waren. Danach wurden nacheinander 38,84 g einer Kleberlösung (Lösung eines selbstklebenden Polymers auf der Basis von Polyacrylsäureestern in Ethylacetat, 51,0 Gew.-%; Durotak^{®} 387-2287), 11,50 g eines Klebrigkeit vermittelnden Harzes (Hercolyn^{®} D), 7,5 g Glycerin sowie 6,0 g einer Lösung von Aluminiumacetylacetonat in Ethylacetat (4 Gew.-%) gegeben und nachfolgend homogenisiert. Die resultierende Masse hatte einen Feststoffgehalt von 45 Gew.-%. Diese Masse wurde sodann auf eine silikonisierte Polyesterfolie (Hostaphan^{®} RN100) mittels Filmziehgerät in einer Schichtdicke von 250 µm aufgetragen und unter definierten Bedingungen getrocknet (30 min bei 50 °C). Der getrocknete Film wurde mit einer flexiblen Polyesterfolie (Hostaphan^{®} RN15) abgedeckt, welche die spätere Rückschicht darstellte. Die Matrix 1b wurde in analoger Weise gefertigt mit dem Unterschied, daß die abschließende Abdeckung mit einer flexiblen Polyesterfolie ausgesetzt wurde. Zur Bereitung der Matrix 2 wurden zuerst 30,0 g eines hochmolekularen Polymers auf der Basis von Methacrylsäureester (Plastoid^{®} B) in Ethylacetat gelöst, so daß eine 30 Gew.-%ige Lösung resultierte. Diese Lösung wurde auf eine silikonisierte Polyesterfolie (Hostaphan^{®} RN100) mittels Filmziehgerät in einer Schichtdicke von 250 µm aufgetragen und unter definierten Bedingungen getrocknet (30 min bei 50 °C).

Nach Vorliegen der getrockneten Matrizes wurde von der Matrix 1a die silikonisierte Polyesterfolie entfernt und auf die Matrix 2 kaschiert. Anschließend wurde die silikonisierte Polyesterfolie von der Matrix 2 entfernt und mit der Matrix 1b kaschiert. Es resultierte ein System, umfassend die Schichten: a) Rückschicht (Hostaphan^{®} RN15), b) Matrix 1a, c) Matrix 2, d) Matrix 1b und e) Schutzfolie (Hostaphan^{®} RN100). Das System ist schematisch in Fig. 1 wiedergegeben.

Zur Beurteilung des kalten Flußes wurde die genannte Formulierung und eine Formulierung, welche nur die Matrizes 1a und 1b enthielt, unter definierten Bedingungen (40 °C, 75 % relativer Feuchte) eingelagert. Das Laminat ohne Matrix 2 zeigte bereits nach einem Monat starken kalten Fluß. Im Gegensatz dazu war ein solcher bei dem Laminat mit Matrix 2 nicht zu beobachten.

## Patentansprüche

1. Wirkstoffhaltiges therapeutisches System zur Applikation auf der Haut, umfassend eine Rückschicht, drei aufeinander laminierte Matrixschichten und eine ablösbare Schutzschicht, **dadurch gekennzeichnet, daß** die mittlere der drei Matrixschichten eine höhere Glasübergangstemperatur hat als die äußeren Matrixschichten.

2. Therapeutisches System nach Anspruch 1, **dadurch gekennzeichnet, daß** sich die für die mittlere Matrixschicht und die für die äußeren Matrixschichten eingesetzten Polymere in ihrer Glasübergangstemperatur unterscheiden.

3. Therapeutisches System nach Anspruch 1 oder 2, **dadurch gekennzeichnet, daß** die äußeren Matrixschichten die gleiche Glasübergangstemperatur haben.

4. Therapeutisches System nach einem der vorangehenden Ansprüche, **dadurch gekennzeichnet, daß** die Zusammensetzung der äußeren Matrixschichten gleich ist.

5. Therapeutisches System nach einem der vorangehenden Ansprüche, **dadurch gekennzeichnet, daß** eine der Schichten ein hochmolekulares Polymer enthält, welches filmbildende Eigenschaften besitzt.

6. Therapeutisches System nach einem der vorangehenden Ansprüche, **dadurch gekennzeichnet, daß** eine der Schichten zugleich als Steuerungsmittel für die Wirkstofffreigabe ausgebildet ist.

7. Verfahren zur Herstellung eines therapeutischen Systems nach einem der vorangehenden Ansprüche, **dadurch gekennzeichnet, daß** drei polymerhaltige Schichten aufeinander laminiert werden, wobei die mittlere Schicht eine höhere Glasübergangstemperatur hat als die beiden äußeren Schichten, und auf eine Quervernetzung verzichtet wurde.

## Claims

1. Active substance-containing therapeutic system for application to the skin, comprising a backing layer, three matrix layers laminated one upon the other, and a detachable protective layer, **characterised in that** the middle one of said matrix layers has a greater glass transition temperature than the outer matrix layers.

2. Therapeutic system according to claim 1, **characterised in that** the polymers used for the middle matrix layer and for the outer matrix layers differ in their glass transition temperature.

3. Therapeutic system according to claim 1 or 2, **characterised in that** the outer matrix layers have the same glass transition temperature.

4. Therapeutic system according to any one of the preceding claims, **characterised in that** the composition of the outer matrix layers is identical.

5. Therapeutic system according to any one of the preceding claims, **characterised in that** one of the layers contains a high-molecular weight polymer having film-forming properties.

6. Therapeutic system according to any one of the preceding claims, **characterised in that** one of the layers is formed to simultaneously serve as a control means for active substance release.

7. Method for the production of a therapeutic system according to any one of the preceding claims, **characterised in that** three polymer-containing layers are laminated one upon the other, the middle layer having a greater glass transition temperature than the two outer layers, and **in that** crosslinking was dispensed with.

## Revendications

1. Système thérapeutique contenant une ou plusieurs substances actives, pour l'application sur la peau, comprenant une couche dorsale, trois couches matricielles stratifiées l'une sur l'autre et une couche de protection amovible, **caractérisé en ce que** la couche médiane parmi les trois couches matricielles possède une température de transition vitreuse supérieure à celle des couches matricielles externes.

2. Système thérapeutique selon la revendication 1, **caractérisé en ce que** les polymères mis en oeuvre pour la couche matricielle médiane et les polymères mis en oeuvre pour les couches matricielles externes se distinguent en ce qui concerne leur température de transition vitreuse.

3. Système thérapeutique selon la revendication 1 ou 2, **caractérisé en ce que** les couches matricielles externes possèdent la même température de transition vitreuse.

4. Système thérapeutique selon l'une quelconque des revendications précédentes, **caractérisé en ce que** la composition des couches matricielles externes est identique.

5. Système thérapeutique selon l'une quelconque des revendications précédentes, **caractérisé en ce qu'**une des couches contient un polymère à poids moléculaire élevé, qui possède des propriétés filmogènes.

6. Système thérapeutique selon l'une quelconque des revendications précédentes, **caractérisé en ce qu'**une des couches est également réalisée pour faire office d'agent de réglage pour la libération de la/des substances actives.

7. Procédé pour la fabrication d'un système thérapeutique selon l'une quelconque des revendications précédentes, **caractérisé en ce qu'**on stratifie l'une sur l'autre trois couches contenant un ou plusieurs polymères, la couche médiane possédant une température de transition vitreuse supérieure à celle des deux couches externes, et on renonce à une réticulation transversale.
